(19) Europäisches Patentamt
European Patent Office
Office européen des brevets

(11) **EP 2 365 337 A1**

(12) **EUROPEAN PATENT APPLICATION**

(43) Date of publication:
**14.09.2011 Bulletin 2011/37**

(51) Int Cl.:
***G01N 33/84*** (2006.01)

(21) Application number: **11157638.5**

(22) Date of filing: **10.03.2011**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **10.03.2010 JP 2010053215**

(71) Applicants:
• **Fujifilm Corporation**
  **Minato-ku**
  **Tokyo (JP)**
• **National University Corporation**
  **Hokkaido University**
  **Sapporo-shi, Hokkaido 060-0808 (JP)**

(72) Inventors:
• **Ogikubo, Shinya**
  **Ashigarakami-gun**
  **Kanagawa-ken (JP)**
• **Adachi, Takashi**
  **Ashigarakami-gun**
  **Kanagawa-ken (JP)**
• **Ohta, Nobuhiro**
  **Sapporo-shi**
  **Hokkaido (JP)**
• **Nakabayashi, Takakazu**
  **Sapporo-shi**
  **Hokkaido (JP)**

(74) Representative: **HOFFMANN EITLE**
**Patent- und Rechtsanwälte**
**Arabellastraße 4**
**81925 München (DE)**

(54) **Intracellular pH imaging method and apparatus using fluorescence lifetime**

(57) The pH of a substance to be measured can be measured without being influenced by the intensity of excitation light (L1), the concentration of fluorescent material and surrounding environments. In a pH measurement method, pulsed excitation light (L1) including a wavelength that can excite a predetermined fluorescent material (P) contained in living matter is generated. The fluorescent material acts as coenzyme in oxidation/reduction reaction in vivo. Further, the intensity of the pulsed excitation light (L1) does not damage a tissue nor a cell in the living matter, and does not substantially change the pH of the living matter. Further, a predetermined position in the living matter is illuminated with the pulsed excitation light (L1), and light including fluorescence emitted from the fluorescent material excited by illumination with the pulsed excitation light (L1) is received. The lifetime (Tf) of the fluorescence is calculated by time-resolving the intensity of the received fluorescence, and the pH of the living matter is measured based on the lifetime (Tf).

**FIG.1**

EP 2 365 337 A1

**Description**

BACKGROUND OF THE INVENTION

Field of the Invention

The present invention relates to a method for measuring the pH of living matter, a method for detecting an abnormal region based on the pH, a method for analyzing the living matter based on the result of measurement and the result of detection, and apparatuses for carrying out these methods.

Description of the Related Art

The values of pH in a cell and a tissue are important factors that regulate functions of an organism (living body), and they are closely related to energy metabolism environment and oxidation/reduction condition in the cell and the tissue. Diseases related to a change in pH in cells and tissues are, for example, cancers, organ hypofunction, and the like.
Cancers and malignant tumors are the leading cause of death in Japan, and most of them are solid cancers, in which cells in organs are cancerized. In treatment of cancers, it is extremely important to detect cancers at early stages and to identify malignant regions. In diagnosis of cancers, pathological examination of tissue, in which a suspicious tissue is extracted from a patient body and judged, is performed.
In the pathological examination of tissue, there are a method for observing a specimen with the naked eye, and a method for performing histological search on the specimen by using an optical microscope. Further, there is a method for biochemically diagnosing the patient by labeling the specimen and by detecting the physical properties of the label by using a microscope. However, since the histological method judges the specimen based on the morphology of a target of pathology, the accuracy of examination heavily relies on the experience of an examiner who performs diagnosis. Meanwhile, in the method of labeling the specimen, the specimen is labeled with a fluorescent dye, and the intensity of fluorescence is detected, or the like. Since the sensitivity of detection is high in the method of labeling the specimen, the method of labeling the specimen is used in many areas.
Japanese Unexamined Patent Publication No. 2000-139462 (Patent Document 1) discloses a method for evaluating drug absorption characteristics of intestine tubes by measuring a change in the pH of a cell labeled with a fluorescent dye based on the intensity of fluorescence from the label. Further, PCT Japanese Publication No. 2001-523334 (Patent Document 2) discloses a method for grouping cells based on fluorescent spectra by labeling the cells with a fluorescent dye. However, since fluorescent dyes are harmful to an organism, there is a risk of damaging a target of measurement. Further, the intensity of fluorescence and the fluorescent spectra change depending on the wavelength and the intensity of excitation light, the concentration of a fluorescent material, fading, and the like. Further, since fluorescence from plural materials and fluorescence from surroundings are reflected in the result of analysis, it is difficult to make highly accurate evaluation.
Meanwhile, fluorescence lifetime is a unique value reflecting the environment of a material. Therefore, the fluorescence lifetime is expected to be useful to accurately observe the environment (condition) of a living matter without being influenced by the condition of excitation light and unnecessary fluorescent material in the surroundings. PCT Japanese Publication No. 2003-512085 (Patent Document 3) discloses a method using at least two spectral techniques in combination. In Patent Document 3, a morphological change and a biochemical change, which are related to a change in living body tissue, are separately evaluated to accurately diagnose a tissue. Patent Document 3 discloses, as spectral techniques for evaluating the biochemical change, a method for evaluating the biochemical change by measuring fluorescence (autofluorescence) by time-resolving fluorescence measurement (fluorescence lifetime measurement) . In Patent Document 3, a fluorescent material that is present in living matter is used as a label, and fluorescence from the fluorescent material by excitation of the fluorescent material is measured.
However, in the method disclosed in Patent Document 3, at least two kinds of evaluation using spectral techniques are necessary to make accurate diagnosis. For easy and quick diagnosis, it is desirable that diagnosis is possible by performing only one kind of evaluation.

SUMMARY OF THE INVENTION

In view of the foregoing circumstances, it is an object of the present invention to provide a pH measurement method for measuring the pH of living matter quickly and at high sensitivity without greatly damaging a target of measurement. Further, it is another obj ect of the present invention to provide a detection method for detecting an abnormal region based on the pH. Further, it is still another object of the present invention to provide a method for analyzing the living matter based on the result of measurement and the result of detection.
Further, it is another object of the present invention to provide apparatuses for carrying out the pH measurement method, the detection method, and the method for analyzing the living matter based on the result of measurement and the result of detection.

A pH measurement method according to the present invention is a pH measurement method comprising the steps of:

generating pulsed excitation light including a wavelength that can excite a predetermined fluorescent material contained in living matter, the fluorescent material acting as coenzyme in oxidation/reduction reaction in vivo, and the intensity of the pulsed excitation light not damaging tissue nor a cell in the living matter and substantially not changing the pH of the living matter;

illuminating a predetermined position in the living matter with the pulsed excitation light;

receiving light including fluorescence emitted from the fluorescent material excited by illumination with the pulsed excitation light;

calculating the lifetime (fluorescence lifetime) of the fluorescence by time-resolving the intensity of the received fluorescence; and

measuring the pH of the living matter based on the lifetime.

Further, a pH measurement apparatus according to the present invention is a pH measurement apparatus comprising:

an excitation light generation means that generates pulsed excitation light including a wavelength that can excite a predetermined fluorescent material contained in living matter, the fluorescent material acting as coenzyme in oxidation/reduction reaction in vivo, and the intensity of the pulsed excitation light not damaging tissue nor a cell in the living matter and substantially not changing the pH of the living matter;

an excitation light illumination means that illuminates a predetermined position in the living matter with the pulsed excitation light;

a light receiving means that receives light including fluorescence emitted from the fluorescent material excited by illumination with the pulsed excitation light;

a time-resolving means that time-resolves the fluorescence synchronously with illumination with the pulsed excitation light;

a detection means that detects the time-resolved fluorescence; and

a measurement means that calculates the Lifetime of the fluorescence based on the time-resolved fluorescence detected by the detection means and measures the pH of the living matter based on the lifetime.

Here, some kind of fluorescent material may be present in a cell in different conditions, such as a protein-bond state and a free state. The state of the fluorescent material may be any state, such as the protein-bond state and the free state, or an average value of a region in a tissue or a cell. Further, the term "living matter" refers to a cell or a tissue of a human or an animal, a biological tissue, such as body fluid, and the like. The living matter maybe any kind of matter including matter extracted from an organism, cultured matter, a cell or tissue in vivo, and the like.

In the pH measurement method and the pH measurement apparatus according to the present invention, it is desirable that the fluorescent material is at least one kind of fluorescent material selected from the group consisting of NADH, NADPH and FAD.

In the pH measurement method according to the present invention, it is desirable that the lifetime of the received fluorescence is calculated for each wavelength by wavelength-resolving the fluorescence to obtain a fluorescence spectrum of the fluorescence and by time-resolving, based on the fluorescence spectrum, the intensity of the fluorescence for the respective wavelengths. Therefore, it is desirable that the pH measurement apparatus according to the present invention includes a light receiving means for obtaining a fluorescence spectrum.

In the pH measurement method according to the present invention, it is desirable that the fluorescence is excited by multi-photon excitation. In such structure, it is possible to easily obtain information about a deep part of living matter by using, as the excitation light generation means, a laser that emits pulses with a pulse width in the range of from a femtosecond to hundreds of picoseconds.

Further, in the pH measurement method and the pH measurement apparatus according to the present invention, the predetermined position may be a plurality of positions. When a detection means for detecting an abnormal region in living matter is provided in the pH measurement apparatus, which is structured as described above, it is possible to detect the abnormal region in living matter.

The abnormal region may be detected by generating and displaying an image of the abnormal region.

Living matter may be analyze by identifying the pathological condition of the living matter based on pH and an abnormal region that are obtained by using the pH measurement method and the detection method according to the present invention.

Optionally, the pH measurement apparatus according to the present invention may be a microscope including:

a stage that keeps the living matter in contact with a surface of the stage, and which is movable in three-dimensional directions so that an arbitrary position in the living matter is illuminated with the pulsed excitation light; and
a position adjustment means that moves the stage to an arbitrary position in three-dimensional directions, wherein the excitation light illumination means includes an optical system that receives the pulsed excitation light and illuminates the living matter with the pulsed excitation light, and
wherein the light receiving means includes an optical system that receives the light including the fluorescence emitted from the fluorescent material excited by illumination with the pulsed excitation light, and that guides the light to the time-resolving means.

Optionally, the pH measurement apparatus according to the present invention may be a pH measurement apparatus including:

a stage that keeps the living matter in contact with a surface of the stage, and which is movable so that an arbitrary position in the living matter, at least in the direction of an optical axis of the pulsed excitation light, is illuminated with the pulsed excitation light;
a first position adjustment means that moves the stage to an arbitrary position at least in the direction of the optical axis; and
a second position adjustment means that moves the excitation light illumination means so that an arbitrary position at least in an in-plane direction, which is perpendicular to the optical axis of the pulsed excitation light, in the living matter is illuminated with the pulsed excitation light,
wherein the excitation light illumination means includes an optical system that receives the pulsed excitation light and illuminates the living matter with the pulsed excitation light, and
wherein the light receiving means includes an optical system that receives the light including the fluorescence emitted from the fluorescent material excited by illumination with the pulsed excitation light, and that guides the light to the time-resolving means.

Optionally, the pH measurement apparatus according to the present invention may be a pH measurement apparatus including:

a position adjustment means that moves the excitation light illumination means so that an arbitrary position in three-dimensional directions in the living matter is illuminated with the pulsed excitation light,
wherein the excitation light illumination means includes an optical system that receives the pulsed excitation light and illuminates the living matter with the pulsed excitation light, and
wherein the light receiving means includes an optical system that receives the light including the fluorescence emitted from the fluorescent material excited by illumination with the pulsed excitation light, and that guides the light to the time-resolving means.

Optionally, the pH measurement apparatus according to the present invention may be a fiber probe in which the excitation light illumination means includes at least one optical fiber for illumination that illuminates the living matter with the pulsed excitation light, and the light receiving means includes at least one optical fiber for receiving light that receives the light including the fluorescence emitted from the fluorescent material excited by illumination with the excitation light and that guides the fluorescence to the time-resolving means. In this embodiment, it is desirable that the at least one optical fiber for illumination and the at least one optical fiber for receiving light form a bundle fiber. Further, it is desirable that the bundle fiber is formed by bundling an optical fiber for illumination and a plurality of optical fibers for receiving light together in such a manner that the outer surface of the optical fiber for illumination arranged substantially at the center of the bundle fiber is surrounded by the plurality of optical fibers for receiving light.

As stated in the section of "Related Art", Patent Document 3 discloses a method for biochemically evaluating a change in a living body tissue by using, as a label, a fluorescent material that is present in living matter. In the method disclosed in Patent Document 3, the change in the living body tissue is evaluated by measuring fluorescence (autofluorescence), which is emitted from the fluorescent material by excitation of the fluorescent material, by time-resolving fluorescence measurement (fluorescence lifetime measurement) . Further, Patent Document 3 describes that pH in a cell is one of intracellular environments that contribute to a change in fluorescence lifetime. However,Patent Document 3 also describes that accurate diagnosis is difficult only by measuring fluorescence lifetime.

The inventors of the present invention found the reason why accurate diagnosis is not possible only by detecting a change in pH by measuring the lifetime of autofluorescence. Further, the inventors of the present invention discovered a method in which pH is accurately measured only by measuring lifetime of of autofluorescence, and in which a change in living body tissue is accurately evaluated based on the measurement result, and an apparatus for realizing the method.

The inventors of the present invention noted that intracellular pH acts as a production factor in production of lactic acid from pyruvic acid. In the reaction of producing lactic acid from pyruvic acid, hydrogen ions are essential, and the concentration of pyruvic acid and the concentration of lactic acid are influenced by the concentration of hydrogen ions, which is represented by the intracellular pH. Several oxidation/reduction reactions occur in production of lactic acid from pyruvic acid, in citric acid cycle starting with acetyl CoA produced from pyruvic acid, and in synthesis of fatty acid from acetyl CoA. In the oxidation/reduction reactions, NADH, NADPH and FAD act as coenzymes. For example, NADH acts as a coenzyme in the process of producing lactic acid from pyruvic acid and in citric acid cycle. Further, NADPH acts as a coenzyme in the process of synthesizing fatty acid from acetyl CoA. Further, FAD acts as a coenzyme in the process of citric acid circuit.

In these processes, dissociation of hydrogen ions from the auto fluorescent material and bond of hydrogen ions to the autoflucrescent material occur by oxidation/reduction reaction, and the absorption spectrum changes. Therefore, there are cases in which the autofluorescent material is not excited depending on the wavelength of excitation light, and no fluorescence is emitted. Further, these materials are present in different conditions, such as a free state and a protein-bond state (please refer to examples that will be described later), in which the fluorescent material is excited by excitation light at the same wavelength but fluorescence having different lengths of fluorescence lifetime is emitted.

Specifically, according to our observation and interpretation, oxidation/reduction reaction is induced by a change in intracellular pH, and dissociation and bond of hydrogen ions of coenzyme occurs. Further, the hydrogen ion concentration that contributes to fluorescence lifetime by a change in the absorption spectrum of the coenzyme change. This change is more prominent in a free state than in a protein-bond state. Therefore, the average fluorescence lifetime of the entire cell changes. Hence, it is possible to accurately evaluate intracellular pH by measuring a change in the fluorescence lifetime of an autofluorescent material that acts as a coenzyme.

Patent Document 3 merely describes that intracellular pH is one of intracellular environments that contribute to a change in fluorescence lifetime. Further, Patent Document 3 does not even suggest presence of desirable autofluorescent material, as clearly seen from the description that accurate evaluation is impossible by evaluating fluorescence lifetime alone.

The inventors of the present invention discovered autofluorescent materials having highly-sensitive fluorescence lifetime, considering the pH of living matter. The present invention succeeded, for the first time, in performing accurate evaluation as to whether a cell is a normal cell or an abnormal cell by using a simple method in which only pH is measured by evaluating fluorescence lifetime.

In, the present invention, the pH of living matter is accurately measured by measuring fluorescence lifetime of an autofluorescent material contained in the living matter, and which acts as coenzyme in oxidatian/reduction reaction in vivo. Accordingly, normal cells and abnormal cells are accurately identified. The fluorescence lifetime is unique to each fluorescent material, and the fluorescence lifetime is not influenced by the intensity of excitation light, the concentration of fluorescent material, and fluorescence from unwanted material. Therefore, the pH of living matter can be measured based on the fluorescence lifetime. Hence, in the present invention, it is possible to highly accurately measure the pH of living matter without being influenced by the intensity of excitation light the concentration of fluorescent material, and fluorescence from unwanted material, and to identify pathological condition as to whether the condition is normal or abnormal.

Further, in the present invention, since the fluorescence lifetime of an auto fluorescent material is a target of measurement, administration of medicine to living matter or the like is not necessary. Therefore, measurement of pH is possible without greatly damaging the living matter.

BRIEF DESCRIPTION OF THE DRAWINGS

Figure is a schematic diagram illustrating the configuration of a pH measurement apparatus, an abnormal region detection, apparatus, and a living matter analysis apparatus according to a first embodiment of the present invention;

Figure 2 is a diagram illustrating a result of average fluorescence lifetime measurement of autofluorescence components having short fluorescence lifetime in an entire image of intracellular pH imaging;

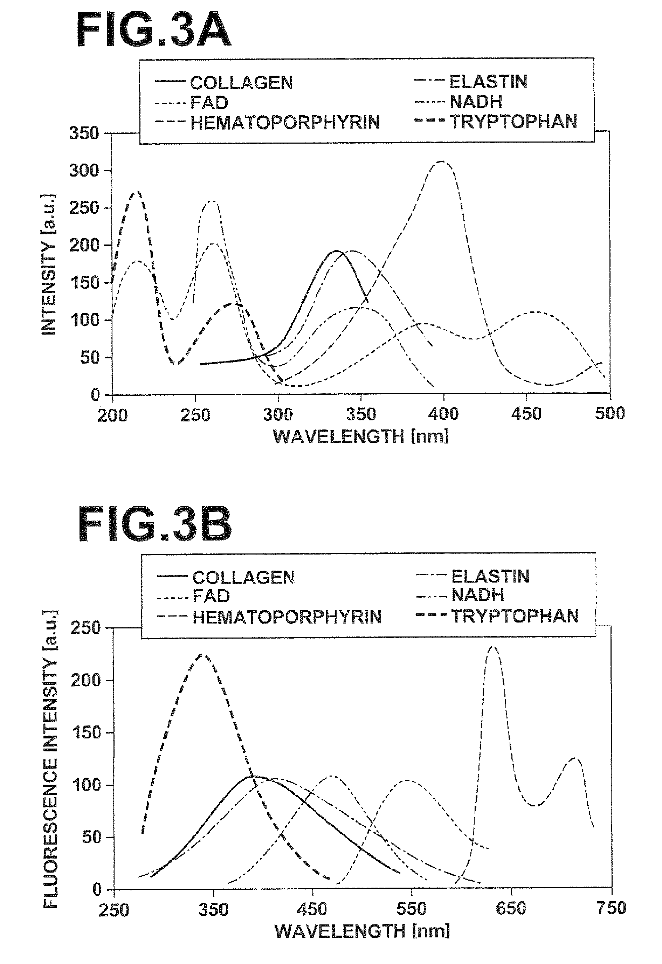

Figure 3A is a diagram illustrating absorption characteristics of excitation light by major autofluorescent materials contained in living matter;

Figure 3B is a diagram illustrating fluorescence characteristics of the autofluorescent materials illustrated in Figure 3A;

Figure 4 is a diagram illustrating the method for calculating fluorescence lifetime by time-resolving;

Figure 5 is a schematic diagram illustrating the configuration of a pH measurement apparatus including a scan means in an optical system of an excitation light illumination means, an abnormal region detection apparatus, and a living matter analysis apparatus;

Figure 6 is a schematic diagram illustrating the configuration of a pH measurement apparatus, in which a spectral element is provided in a light receiving means in the pH measurement apparatus illustrated in Figure 5, an abnormal region detection apparatus, and a living matter analysis apparatus;

Figure 7 is a schematic diagram illustrating the configuration of a pH measurement apparatus, an abnormal region detection apparatus, and a living matter analysis apparatus according to a second embodiment of the present invention;

Figure 8A is a schematic diagram illustrating the structure of an endoscope when a fiber bundle is provided in the endoscope;

Figure 8B is a schematic diagram illustrating the structure of an endoscope when a fiber probe is provided in the endoscope;

Figure 9A is a diagram illustrating a result of fluorescence intensity imaging of HeLa cell in Example 1; and

Figure 9B is a diagram illustrating a result of fluorescence intensity imaging of HeLa cell in Example 1.

DESCRIPTION OF THE PREFERRED EMBODIMENT

"First Embodiment of pH Measurement Apparatus (Microscope)"

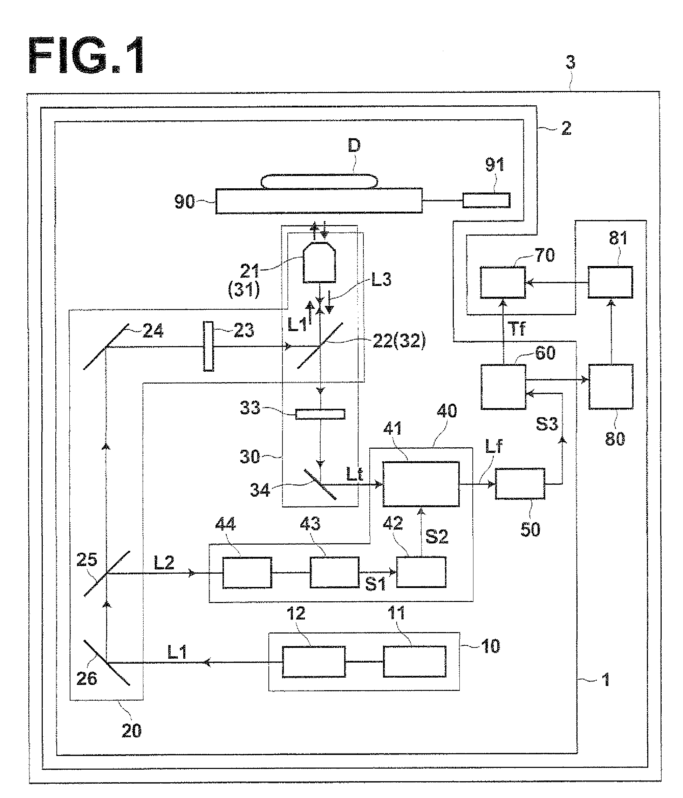

A pH measurement apparatus and a pH measurement method according to a first embodiments of the present invention will be described with reference to drawings. Figure 1 is a schematic diagram illustrating the configuration of a pH measurement apparatus 1 according to an embodiment of the present invention. In Figure 1, each unit is illustrated at an appropriate scale so as to be easily recognized.

As illustrated in Figure 1, the pH measurement apparatus 1 outputs pulsed excitation light L1 (hereinafter, referred to as excitation light L1) to substance D to be measured, which is living matter, by an excitation light illumination means 20. Further, the pH measurement apparatus 1 obtains fluorescence by receiving light L3 by a light receiving means 30. The light L3 includes fluorescence Lf emitted, by illumination with the excitation light L1, from fluorescent material P contained in the substance D to be measured. Further, a time-resolving means 40 time-resolves the fluorescence into predetermined fluorescence Lf, and a detection means 50 detects the time-resolved fluorescence Lf. Further, a measurement means 60 obtains a fluorescence decay curve based on the intensity of the detected fluorescence Lf, and calculates lifetime TF of the fluorescence Lf. Further, the pH of the substance D to be measured is measured based on the lifetime Tf. The excitation light L1 includes a wavelength that can excite predetermined fluorescent material P contained in living matter in the substance D to be measured, the fluorescent material acting as coenzyme in oxidation/reduction reaction in vivo. Further, the intensity of the pulsed excitation light L1 is selected in such a manner that a tissue nor a cell in the living matter is not damaged and the pH in the living matter is not substantially changed.

The method for measuring the fluorescence lifetime is basically divided into a time domain measurement method and a frequency domain measurement method. In the present embodiment, the time domain measurement method is adopted, Examples of the time domain measurement method are a streak camera method, a time correlated single photon counting method, a time gate method, and the like. In the present embodiment, a case of measuring fluorescence lifetime by using the time gate method will be described.

In the time gate method, emitted fluorescence is divided to a certain time domain synchronously with output of pulsed excitation light. Further, the integral value of fluorescence intensity in the range of the time domain is detected by a solid-state detector, such as a CCD, and measurement is performed for a different time domain. Accordingly, a fluorescence decay curve is approximated. In the present embodiment, when signal light L2 separated from the excitation light L1 by a beam splitter 25 is detected by a photodiode 44, a synchronization control apparatus 43 sends synchronization signal S1 to a gate controller 42. When the gate controller 42 receives the synchronization signal S1, the gate controller 42 originates gate signal S2 in a predetermined time domain that has been set in advance, and an image intensifier 41 with a gate function is driven. Fluorescent image Lf that has been input to the image intensifier 41 with a gate function is intensified in a time domain corresponding to the gate signal S2. Further, The detection means 50 detects the integral value (fluorescent signal) Lt of the fluorescence intensity of the predetermined fluorescence Lf. This process is repeated based on the gate signal S2. Further, the measurement means 60 calculates fluorescence lifetime Tf by performing analysis by a single exponential function.

The inventors of the present invention measured fluorescence lifetime of various autofluorescent materials several times by using the time correlated single photon counting method (TCSPC) and by the time gate method, and discovered that a marginal value that can separate (distinguish) two different fluorescence Lifetimes from each other is 0.03 nsec in the current fluorescence lifetime measurement technique. In the pH measurement apparatus of the present embodiment, an auto fluorescent material, the fluorescence lifetime of which changes by at least 0.03 nsec as pH changes reflecting whether a cell is a normal cell or an abnormal cell, is excited, and the fluorescence lifetime is measured. Accordingly, it is possible to highly accurately identify whether living matter is normal or abnormal by measuring the fluorescence lifetime.

As state above already, intracellular pH acts as a production factor that produces lactic acid from pyruvic acid, and the concentration of pyruvic acid and the concentration of lactic acid are influenced by the concentration of hydrogen ions, which is represented by the intracellular pH. In production of lactic acid from pyruvic acid, citric acid cycle starting with acetyl CoA produced from pyruvic acid, and synthesis of fatty acid from acetyl CoA, several oxidation/reduction reactions occur. In the oxidation/reduction reactions, NADH, NADPH and FAD act as coenzymes. For example, NADH acts as a coenzyme in the process of producing lactic acid from pyruvic acid and in citric acid cycle. NADPH acts as a coenzyme in the process of synthesizing fatty acid from acetyl CoA. FAD acts as a coenzyme in the process of citric acid circuit.

In these processes, dissociation of hydrogen ions from the autofluorescent material and bond of hydrogen ions to the autofluorescent material occur by oxidation/reduction reaction, and the absorption spectrum changes. Therefore, there are cases in which fluorescence is not excited depending on the wavelength of excitation light, and no fluorescence is emitted. Further, these materials are present in different conditions, such as a free state and a protein-bond state, in which the fluorescent material is excited by excitation light at the same wavelength but fluorescence having different lengths of fluorescence lifetime is emitted (please refer to examples that will be described later).

The inventors of the present invention think axidation/reduction reaction is induced by a change in intracellular pH, and dissociation and bond of hydrogen ions of coenzyme occurs by the oxidation/reduction reaction. Therefore, the concentration of hydrogen ions that contributes to fluorescence lifetime by a change in the absorption spectrum of the coenzyme changes. They think since this change is more prominent in the free state than in the protein-bond state, the average fluorescence lifetime of the entire cell changes. Accordingly, they have discovered that it is possible to accurately evaluate intracellular pH by measuring a change in the fluorescence lifetime of the auto fluorescent material that acts as a coenzyme.

The pH of a cultured HeLa cell was measured by using a microscope-type pH measurement apparatus and an abnormal region detection apparatus illustrated in Figure 1.

First, HeLa cells at different pH values were prepared, and relationships between the pH of the HeLa cells and fluorescence lifetimes were obtained. A HeLa cell at a predetermined pH value was prepared by using, as culture medium, D-MEM (Dulbecco's Modified Eagle Medium, STIGMA Co.). An appropriate amount of HeLa cells was seeded into a quartz glass bottom dish, and cultured at 37°C for one or two days in an incubator with 5% $CO_2$. After then, an appropriate amount of nigericin was added to KCl-rich medium the pH of which had been adjusted by using 0.1 mol/L NaOH solution or HCl solution, and the D-MEM was replaced by KCl-rich medium. In this manner, the HeLa cell at the predetermined pH was prepared. Nigericin is a pH decrease inhibitor for endosome, and acts as an ionophore of K+/H+. Nigericin makes intracellular pH and extracellular pH the same. After the pH values of the HeLa cells were checked as to whether they are intended values, the HeLa cells were placed in an incubator (37°C, 5% $CO_2$) set on an xyz stage of a microscope for 15 minutes. Accordingly, plural samples to be measured having different pH values were prepared.

Further, the fluorescence lifetimes of these samples were measured. Here, NADH and NADPH were used as autofluorescent material to be excited, and SHG light of a 1-picasecond pulse-width titanium sapphire laser was used as excitation light. Further, the wavelength of the excitation light was adjusted to 370 nm, which is an excitation wavelength of NADH and NADPH (only reduction type is excited) . Further, a fluorescent filter of 417 through 477 nm was used, and fluorescence lifetimes were measured. At this time, the average power of the excitation light was 0.9 mW, and pulse energy was 12 pJ. A laser beam was output to a predetermined position in a HeLa cell, and the position of the xyz stage was adjusted so that the laser light is focused on the HeLa cell. The measurement temperature was 23 degrees.

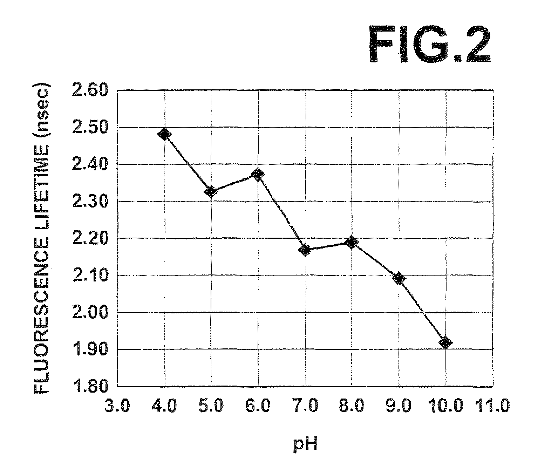

Figure 2 illustrates the obtained result. In Figure 2, the vertical axis represents average fluorescence lifetime in an entire image of intracellular pH imaging. With respect to each pH, samples were changed, and measurement was performed plural times, and the average value was obtained. Further, the following relational equation (1) was obtained by performing fitting on the line illustrated in Figure 2 by using a least square method:

$$[pH] = 33.3 - 11.9 \times [\text{Fluorescence Lifetime (nsec.)}] \qquad (1).$$

Further, as illustrated in Figure 2, the fluorescence lifetime of NADH and NADPH changes by 0.08 nsec in the range of from pH 6.5 to pH 7.5. Meanwhile, when the marginal value of measurement of fluorescence lifetime, which has been described already, is considered, a difference in pH in the range of from 0.3 to 0.4 is measurable. Therefore, the high sensitivity of NADH and NADPH was confirmed.

As these results show, the inventors of the present invention discovered that the pH of living matter can be accurately measured by measuring fluorescence lifetime of an autofluorescent material contained in living matter, and which acts as coenzyme in oxidation/reduction reaction in vivo. Therefore, it is possible to highly accurately measure the pH of living matter without being influenced by the intensity of excitation light, the concentration of fluorescent material, and fluorescence from unwanted material, and to identify pathological condition as to whether the living matter is normal or abnormal.

In a cell, NADH, NADPH and FAD are present mainly in cytoplasm or mitochondria. Therefore, when the region of the cytoplasm or mitochondria is measured, it is possible to analyze intracellular pH, and that is desirable. Meanwhile, the amount of NADH, NADPH and FAD in a nucleus is small. However, since NADH, NADPH and FAD in the vicinity of the nucleus emit autofluorescence, the region of the nucleus may be measured.

The pH measurement apparatus 1 is a confocal microscope, and substance D to be measured is placed on a sample stage 90, which is movable in the directions of x, y and z by a position adjustment means 91. The position of the sample stage 90 is adjusted by the position adjustment means 91 to illuminate a predetermined position in the substance D to be measured with excitation light L1 that has been condensed by an objective lens (optical system) 21 (31). The pH measurement apparatus 1 may include a window (not illustrated) including a magnification lens. The window is provided to check, from the upper side of the substance D to be measured, the illumination position of the excitation light L1 on the substance D to be measured, thereby a predetermined position is accurately illuminated with the excitation light L1.

In the pH measurement apparatus 1, an excitation light generation means 10 includes a pulse laser 11 and a pulse picker 12. The pulse laser 11 generates pulsed excitation light including a wavelength that can excite a predetermined fluorescent material contained in living matter in the substance D to be measured, the fluorescent material acting as coenzyme in oxidation/reduction reaction in vivo, and the intensity of the pulsed excitation light not damaging a tissue nor a cell in the living matter and substantially not changing the pH of the living matter. The pulse picker 12 picks (thins) pulses oscillated by the pulse laser 11 so that pulses are output with predetermined intervals. The pulse laser 11 is not particularly limited, but desirably a laser that can oscillate ultra-short pulsed light. It is more desirable that the pulse laser 11 can oscillate pulses in the time range of from some tens of femtoseconds to some tens of picoseconds. For example, the pulse laser 11 that has the wavelength of 420 nm, a pulse width of 3 ps, a repetition frequency of 80 MHz, a peak power of 8 kW, an average power of 2 W, and the like may be used. The wavelength of 42Q nm is a second harmonics (SHG: second harmonic generation) of a titanium sapphire laser having an oscillation wavelength of 840 nm. Further, the excitation light generation means 10 may include a third harmonic (THG: third harmonic generation) or a wavelength conversion element, such as an optical parametric oscillator (OPO). In the present embodiment, the pulse picker 12 is used, but it is not necessary that the pulse picker 12 is provided. Instead, a light source that outputs light that has been adjusted to a predetermined repetition frequency may be used.

The fluorescence Lf may be excited in the substance D to be measured by multi-photon excitation. In that case, a titanium sapphire laser having an oscillation wavelength of 840 nm, a pulse width of 100 fs, a repetition frequency of 80 MHz, a peak power of 100 kW, an average power of 0.8 W and the like are used as excitation light L1 having a wavelength and a pulse width that can induce multi-photon excitation. In multi-photon excitation, it is possible to excite only a desirable position. Therefore, it is possible to reduce fluorescence in the vicinity of the position, and to further reduce noise. Further, since it is possible to reduce absorption decay before the observation position, it is possible to efficiently illuminate the observation position with the excitation light L1. Further, it is possible to analyze at high spatial resolution.

The excitation light L1 output from the excitation light generation means 10 enters the excitation light illumination means 20. Further, the excitation light illumination means 20 illuminates a predetermined position, in the substance D to be measured with the excitation light L1. The structure of the excitation light illumination means 20 is not particularly limited. However, in the excitation light illumination means 20 of the present embodiment, the same objective lens (optical system) 21 (31) is used to output the excitation light L1 and to receive light L3 including fluorescence Lf emitted from the substance D to be measured by excitation of the fluorescent material P. Therefore, the excitation light illumination means 20 includes the objective lens 21 and a dichroic mirror 22 (32). The objective lens 21 is arranged so that a predetermined position in the substance D to be measured is illuminated with the excitation light L1 and that the light L3 is receivable. The dichroic mirror 22 (32) makes the excitation light L1 enter the objective lens 21, and guides the light L3 to the time-resolving means 40. It is desirable that the material of the objective lens 21 has high transmittance (transmission) with respect to the excitation light L1 and fluorescence. Further, it is desirable that the amount of fluorescence emitted from the lens material is small.

In the present embodiment, the time-resolving means 40 is driven synchronously with output of the excitation light L1. Therefore, the excitation light illumination means 20 includes a beam splitter 25 and an excitation filter 23. The beam splitter 25 separates, from the excitation light L1, signal light L2 that originates synchronization signal S1. The excitation filter 23 filters the excitation light L1 so that light having an wavelength that can excite the fluorescent material P remains. The excitation light illumination means 20 may include a mirror (24, 26), a lens (not illustrated), and the like for guiding the excitation light L1, if necessary.

The light receiving means 30 is not particularly limited as long as it can receive the light L3 emitted from the substance D to be measured by illumination with the excitation light L1, and output intended fluorescence Lf to the time-resolving means 40. However, in the present embodiment, the light receiving means 30 includes an objective lens 31 (21), a dichroic mirror 32 (22), and a filter 33. The objective lens 31 (21) receives light L3 emitted from the substance D to be measured. The dichroic mirror 32 (22) guides the light L3 to the time-resolving means 40, and the filter 33 removes noise light, such as scattered light, from the light L3.

The objective lens (optical system) 31 and the dichroic mirror, 32 are as described above. A filter 34, such as a band-pass filter or a short-pass filter, may be appropriately used based on the wavelength of fluorescence Lf to be detected. When plural kinds of fluorescence Lf need to be observed, plural band-pass filters that pass light of different wavelength bands may be used. Alternatively, a filter that has a variable passing wavelength band may be used.

The fluorescence Lf received by the light receiving means 30 is input to the time-resolving means 40. The time-resolving means 40 extracts fluorescent signal Lt in a predetermined time domain from the fluorescence Lf, and outputs the extracted fluorescent signal Lt to the detection means 50. Time-resolving of fluorescence starts from time to when the first synchronization signal S1 for output of the excitation light L1 is received. The synchronization signal S1 is generated at an optical detector 44 by converting, to an electrical signal, the signal light L2 separated from the excitation light L1 by the beam splitter 25. Further, the synchronization signal S1 is sent from a synchronization control apparatus 43 to a gate controller 42.

The fluorescence Lf received by the light receiving means 30 is input to the time-resolving means 40. The time-resolving means 40 extracts a fluorescent image in a predetermined time domain from predetermined fluorescence Lf, and outputs the extracted fluorescent image to the detection means 50. Time-resolving of fluorescence starts from time t0 when the first synchronization signal S1 for output of the excitation light L1 is received. The synchronization signal S1 is generated at an optical detector 44 by converting, to an electrical signal, the signal light L2 separated from the excitation light L1 by the beam splitter 25. Further, the synchronization signal S1 is sent from a synchronization control apparatus 43 to a gate controller 42.

The light detector 44 is not particularly limited. However, it is desirable to use a photodiode or the like, which is generally used.

The synchronization control apparatus 43 is not particularly limited as long as it can receive a signal output from the light detector 44 and output synchronization signal S1 to the gate controller 42. For example, a personal computer (PC) or the like may be used as the synchronization control apparatus 43. The gate controller 42 receives the synchronization signal S1 and originates gate signal S2 in a predetermined time domain that has been set in advance. Accordingly, the gate controller 42 instructs opening/closing of a gate in an image intensifier 41 with a gate function. A detection means 50, such as a CCD camera, is connected to the image intensifier 41 with a gate function and the gate is opened or closed based on the gate signal S2. Fluorescence signal Lt that has been received while the gate is open is amplified, and fluorescent image Ls, the contrast of which has been improved, is received by the detection means 50, such as a CCD and PMT (photo multiplier tube). Further, the fluorescent image Ls is stored as an electrical signal, and detected. For example, when time when the gate controller 42 has received the synchronization signal S1 for the first time is base time t0, if gate signal S2 for instructing the image intensifier 41 with a gate function to open the gate only for time $\Delta t$ is output, the image intensifier 41 with the gate function amplifies the fluorescent signal Lt in a period from time t0 to 8t, and outputs the amplified signal to the detection means 50. Further, the detection means 50 detects fluorescence Lf in the time range, and outputs the integral value S3 of the intensity of fluorescence to a measurement means 60. This process of detecting the fluorescence intensity S3 is repeated based on the gate signal, and time-resolved fluorescence intensity is output to the measurement means 60.

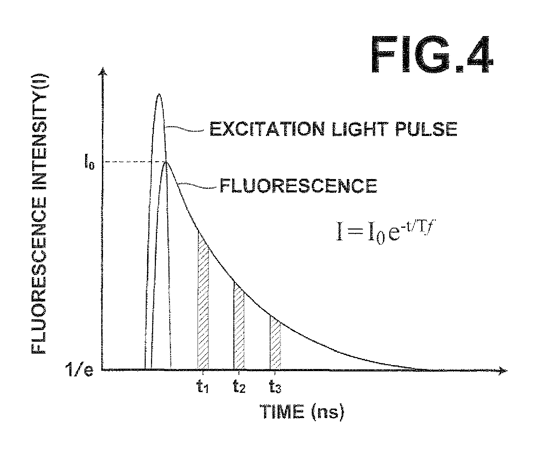

The measurement means 60 is not particularly limited. However, it is easy and desirable to use a computer system), such as a PC, as the detection means 60. The same apparatus may be used as the measurement means 60 and the synchronization control apparatus 43. The measurement means 60 obtains fluorescence intensity time distribution by obtaining a fluorescence decay curve based on the time-resolved fluorescence intensity S3. In the fluorescence decay curve, a difference between time tf and time t0 is calculated as fluorescence lifetime Tf. The time tf is time when the fluorescence intensity becomes 1/e, and the time t0 is time when emission of fluorescence starts. Figure 4 is a diagram illustrating fluorescence intensity time distribution obtained based on time-resolved fluorescence intensity and fluorescence lifetime Tf obtained from the fluorescence decay curve. In Figure 4, fluorescence intensity I when time period t has passed from time t0 and fluorescence intensity $I_o$ at time t0 have a relationship represented by the following formula:

$$I = I_0 e^{-t/Tf}.$$

In the analysis means 60, S3 is fluorescence intensity I at certain time t.

Next, the measurement means 60 measures pH at a predetermined position in the substance D to be measured based on the obtained fluorescence lifetime If. It is known that the fluorescence lifetime of autofluorescent material changes depending on pH in the vicinity of the autofluorescent material.

Therefore, if the fluorescence lifetime of substance D to be measured in normal condition is obtained in advance, it is possible to measure a change in pH at a predetermined position in the substance D to be measured based on the fluorescence lifetime of autofluorescent material at the predetermined position. Further, if a rate of change in fluorescence lifetime with respect to pH is obtained in advance, it is possible to measure a specific pH value. In analysis of pH using fluorescence lifetime imaging by using the time gate method, it is possible to improve the accuracy of analysis by increasing the number of gates.

In the pH measurement apparatus 1, the stage 90 is movable in the directions of x, y and z (in-plane direction and depth direction of an excitation light illumination plane). Therefore, two-dimensional or three-dimensional measurement of pH is possible at predetermined plural positions of the substance D to be measured.

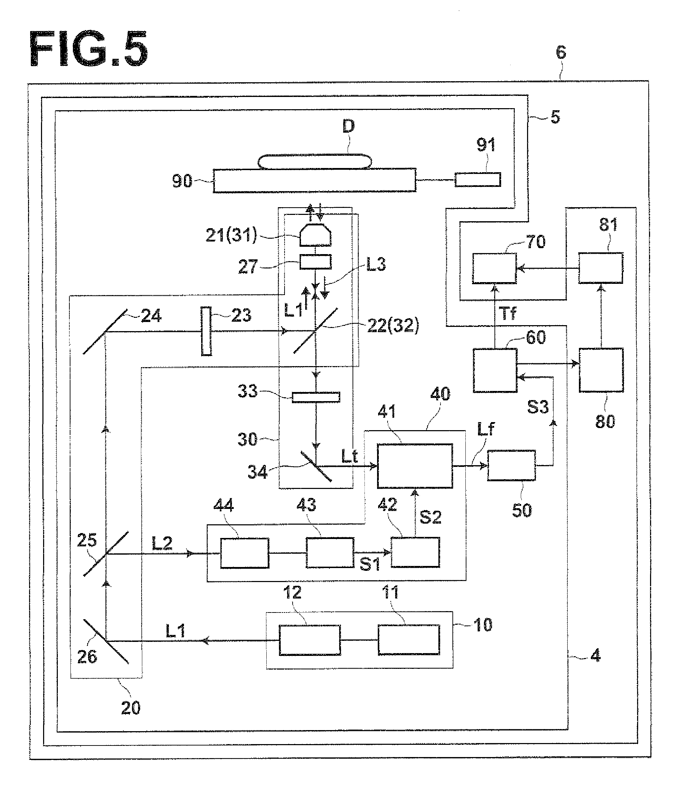

Further, as in a pH measurement apparatus 4 illustrated in Figure 5, a stage 90' maybe movable only in z direction, and a confocal optical system may be adopted as a scan optical system 27. The confocal optical system scans a target in xy direction. Examples of the scan optical system 27 are a galvanomirror, a polygon mirror, a resonant mirror, and the like.

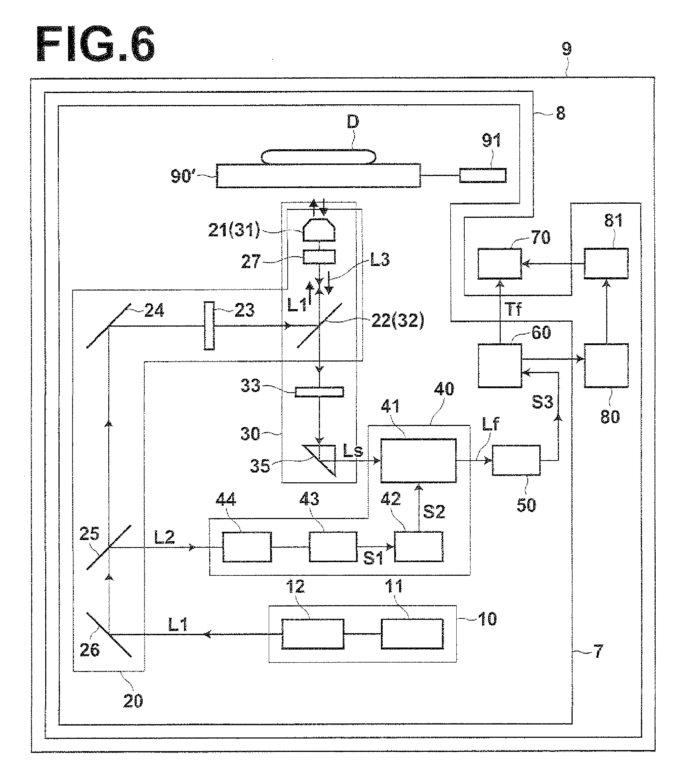

When the pH measurement apparatus is structured in such a manner, there is a high possibility that plural kinds of fluorescence are excited by excitation light L1. Therefore, as in a pH measurement apparatus 7 illustrated in Figure 6, it is desirable that a spectral means 35 is provided. The spectral means 35 obtains fluorescent spectrum Ls of fluorescence Lf received by the light receiving means 30, and outputs the fluorescent spectrum Ls of the fluorescence Lf to the time-resolving means 40. Since the fluorescent spectrum Ls of the fluorescence Lf is obtained, and the fluorescent spectrum Ls is time-resolved, it is possible to more accurately divide the plural kinds of fluorescence from each other, and to accurately measure the lifetime of fluorescence of a predetermined wavelength. The spectral means 35 is not particularly limited as long as a spectrum of the fluorescence Lf is obtainable, and for examples, a diffraction grating, a prism or the like may be used.

The structure including the scan optical system, as illustrated in Figure 5 or 6, is especially desirable when multi-photon excitation that provides localized excitation is performed.

A method for analyzing an abnormal region of the substance D to be measured by measuring pH at plural positions will be described later.

The pH measurement apparatuses 1, 4 and 7 according to the embodiments of the present invention are structured as described above.

Next, an example of operations of a pH measurement apparatus according to an embodiment of the present invention will be described by using the pH measurement apparatus 1 as an example.

First, substance D to be measured is placed on the stage 90, specifically in contact with a surface of the stage 90, and the position of the stage 90 is adjusted by the position adjustment means 91 so that a predetermined position is illuminated with excitation light L1.

The excitation light generation means 10 outputs pulsed excitation light L1 including a wavelength that can excite a predetermined fluorescent material contained in living matter, the fluorescent material acting as coenzyme in oxidation/reduction reaction in vivo, and the intensity of the pulsed excitation light not damaging a tissue nor a cell in the living matter and substantially not changing the pH of the living matter. The pulsed excitation light L1 enters the excitation light illumination means 20. Signal light L2 for synchronization is separated, by the beam splitter 25, from the pulsed excitation light L1 that has entered the excitation light illumination means 20, and the pulsed excitation light L1 is condensed by the objective lens (optical system) 21. Further, a predetermined position in the substance D to be measured is illuminated with the excitation light L1. When the substance D to be measured is illuminated with the excitation light L1, fluorescent material P is excited, and fluorescence Lf is emitted from the fluorescent material P. The objective lens 31 of the light receiving means 30 receives light L3 output from the substance D to be measured. The light L3 includes noise light other than the fluorescence Lf. However, light other than the fluorescence Lf is substantially removed by passing the light $L_3$ through the filter 34.

The light L3 that includes substantially only fluorescence enters the time-resolving means 40, and predetermined fluorescence Lf is extracted, and time-resolved. Further, the detection means 50 detects fluorescence intensity S3 at each time, and the detected fluorescence intensity 53 is output to the measurement means 60 as an electrical signal. The measurement means 60 obtains a fluorescence intensity - time curve based on the time-resolved fluorescence intensity. Further, a difference between time tf and time t0 is calculated as fluorescence lifetime Tf by using the fluorescence intensity - time curve. The time tf is time when the fluorescence intensity substantially becomes 1/e. Further, the pH of the substance D to be measured is measured based on the fluorescence lifetime Tf.

As described above, in the pH measurement method and the pH measurement apparatuses 1, 4 and 7 according to embodiments of the present invention, pH of living matter is highly accurately measured by measuring fluorescence lifetime of an autofluorescent material that acts as coenzyme in oxidation/reduction reaction in vivo, and which is contained in living matter. Further, it is possible to highly accurately identify a normal cell and an abnormal cell. Since the fluorescence lifetime is unique to a material, it is possible to measure the pH of living matter without being influenced by the intensity of excitation light, the concentration of fluorescent material, and fluorescence from unwanted material. In the present invention, the pH of living matter is highly accurately measured without being influenced by the intensity of excitation light, the concentration of fluorescent material, and fluorescence from unwanted material. Further, it is possible to accurately identify pathological condition as to whether the living matter is normal or abnormal.

Further, in the embodiments of the present invention, the fluorescence lifetime of autofluorescent material is a target of measurement. Therefore, it is not necessary to administer a drug or the like to living matter. Hence, measurement of pH is possible without greatly damaging the living matter.

The advantageous effects of the pH measurement apparatuses 1, 4 and 7, as described above, are achievable also by pH measurement apparatuses in other embodiments that will be described later.

So far, a case of using a confocal microscope method has been described. As described above, in the confocal microscope method, the spot diameter of excitation light illuminating the substance to be measured is reduced (narrowed), and fluorescence lifetime in a very small region is measured. Further, a measurement position is shifted to perform measurement on a measurement target region of the substance to be measured. However, when it is necessary to generate an image of a wide area depending on the substance to be measured, a wide field method may be used. In the wide field method, the spot diameter of the excitation is not reduced, and information about a wide area of xy plane is obtained at once. Further, the entire area of a screen may be image by using, as the detection means, a CCD camera or the like without using the scan means. Further, in this case, if the stage 90 (90') can be scanned in xy direction, an image of a wider area is obtainable by detecting and combining plural xy plane areas.

"Second Embodiment of pH Measurement Apparatus (Fiber Probe)"

Next, a pH measurement apparatus according to another embodiment of the present invention will be described. The pH measurement apparatuses 1, 4 and 7 in the first embodiment are microscopes. However, a pH measurement apparatus 10 in the second embodiment is a fiber probe. In the descriptions of the present embodiment and the drawings, the same signs will be assigned to elements that have substantially the same functions as those of the first embodiment, and explanations will be omitted.

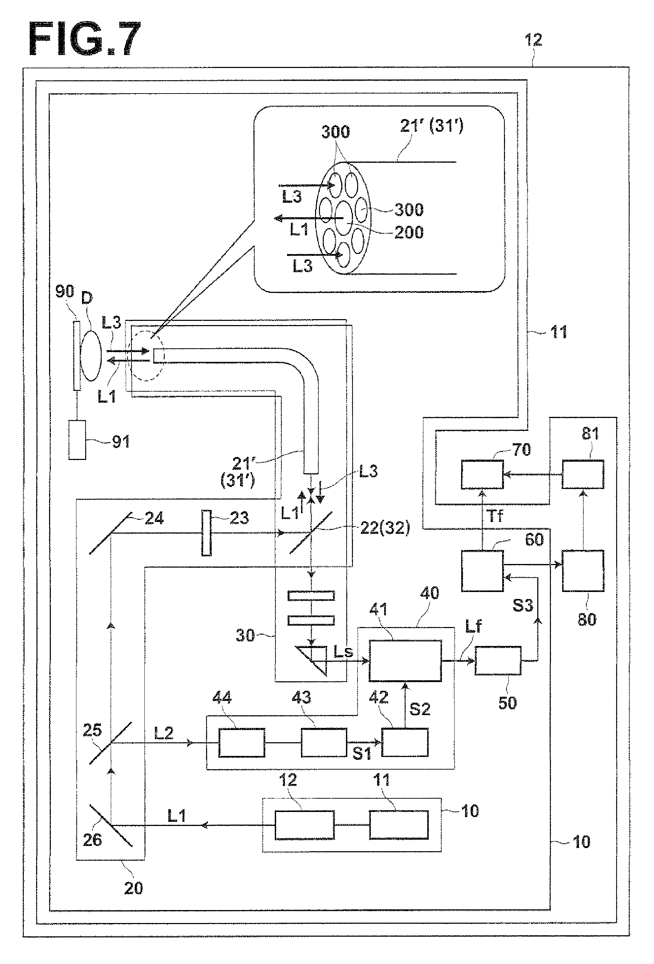

As illustrated in Figure 7, the structure of the pH measurement apparatus 10 is similar to the structure of Embodiment 1, except that the objective lens 21 (31) in the first embodiment is replaced by a bundle fiber 21' (31') in the second embodiment.

In the bundle fiber 21' (31'), an optical fiber 200 for illumination is arranged substantially at the center. The optical fiber 200 for illumination guides the pulsed excitation L1 toward a central part, and illuminates a predetermined position in the substance D to be measured with the excitation light L1. Further, plural optical fibers 300 for receiving light are arranged so as to surround the outer surface of the optical fiber 200 for illumination. The optical fibers 300 for receiving light receive light L3 including fluorescence Lf emitted, by illumination with the excitation light L1, from autofluorescent material P that acts as coenzyme in oxidation/reduction reaction in vivo, and which is contained in the substance D to be measured. Further, the optical fibers 300 for receiving light guide the light L3 to the time-resolving means 40. In Figure 7, seven optical fibers 300 for receiving light are illustrated. However, it is not necessary that the number of the optical fibers 300 for receiving light is seven. At least one optical fiber for illumination and at least one optical fiber for receiving light should be provided, and the number of fibers and the fiber diameters may be appropriately selected based on receivable fluorescence intensity or the like.

The bundle fiber 21' (31') is produced by fixing the optical fiber 200 for illumination, and the optical fiber or fibers 300 for receiving light together by using an adhesive or the like so that they are arranged at predetermined positions. It is desirable to use an additive having a heat resistance temperature appropriate for the excitation L1 used in Measurement, When a heat resistance temperature exceeding 300°C is required, an inorganic heat-resistant additive is used. Further, the fixed bundle fiber may be used as a fiber probe (not illustrated) by providing the bundle fiber in a long and substantially cylindrical sheath to be inserted into body cavity.

The optical fiber 200 for illumination and the optical fibers 300 for receiving light are not particularly limited. However, it is desirable that the material of the optical fiber 200 for illumination and the optical fibers 300 for receiving light has excellent transmittance of the excitation light L1, and is not damaged by the excitation light L1 . Especially, when the excitation light L1 is ultraviolet light or short wavelength light close to the ultraviolet light, the light energy of the excitation light L1 is high. Therefore, in that case, it is desirable to use quartz-based optical fibers.

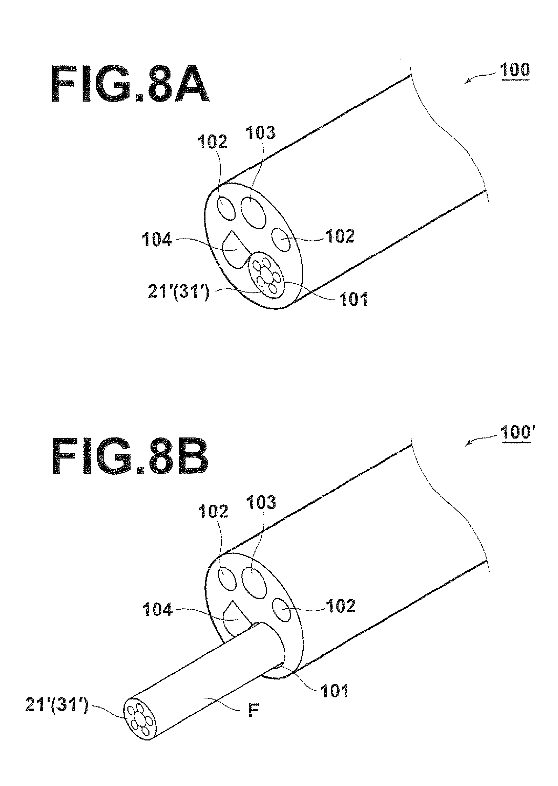

In the second embodiment, it is possible to illuminate the substance D to be measured with the excitation light L1 and to receive light L3 including fluorescence Lf by using fiber probe F. Therefore, measurement in a living body, such as measurement in vivo, is possible. Further, a predetermined position in the substance D to be measured may be determined by an examiner (a user or doctor who performs measurement) by directly moving the probe during examination. For example, as illustrated in Figures 8A and 8B, the bundle fiber 21' (31') may be provided in a forceps channel 101 in an endoscope 100. Alternatively, the fiber probe F may be inserted into the forceps channel 101 in the endoscope 100. In this case, as illustrated in Figures 8A and 8B, the endoscope 100 includes an illumination unit 102, an imaging unit 103, and an air/water supply nozzle 104. The illumination unit 102 illuminates a predetermined position in the body cavity with illumination light. The imaging unit 103 images reflection light L4 reflected at a predetermined position. The illumination unit 102 is connected to a light source that can illuminate a predetermined position. The imaging unit 103 is connected to a monitor (not illustrated) or the like that can make the examiner recognize or check the predetermined position.

In the second embodiment, it is possible to directly measure the pH of tissue of an animal and a human. For example, the pH measurement apparatus according to the second embodiment is appropriate for examination of human digestive organs, tracheas and the like.

"Abnormal Region Detection Apparatus (Detection Apparatus)"

With reference to Figures 1, 5 and 6, abnormal region detection apparatuses (detection apparatuses) 2, 5 and 8 of the present embodiment will be described.

As already described, in the pH measurement apparatus 1 (4, 7), the stage 90 (90') is movable in xy direction (in-plane direction of the excitation light illumination plane) and z direction (the direction of the optical axis). Therefore, it is possible to measure pH at predetermined plural positions in the substance D to be measured. Therefore, for example, when the predetermined plural positions are evenly set in the xy plane of the substance D to be measured, it is possible to obtain in-plane pH distribution of the substance D to be measured. Further, when multi-photon excitation, such as two photon fluorescence, is used, it is possible to detect fluorescence at a deep part of the substance to be measured. In that case, it is possible to obtain three-dimensional pH distribution by setting measurement positions also in the z direction of the substance D to be measured.

Further, when a pH measurement apparatus of a wide field method is used, it is possible to obtain information on xy plane at once, as already described in the embodiment of the pH measurement apparatus. Therefore, it is also possible to easily obtain in-plane pH distribution of the substance D to be measured in a similar manner.

Therefore, when an abnormal region detection means 80 that can detect pH distribution of the substance D to be measured is provided for the pH measurement apparatuses 1, 4 and 7, it is possible to use them, for example, as abnormal region detection apparatuses 2 (5, 8) for detecting an abnormal region.

As the abnormal region detection means, an array detector, such as a CCD and a cooling CCD, may be used.

When the abnormal region detection apparatus 2 (2') further includes a display means 81 that generates and displays an image of a detection result obtained by the abnormal region detection means 80, it is possible to clearly recognize in-plane pH distribution and an abnormal region. As the method for generating an image, a conventional method may be used. For example, a method of displaying in-plane pH distribution by using different colors, a method of three-dimensionally displaying in-plane concentration distribution, and the like may be used. When plural images are obtained, they may be combined and displayed.

Further, when the pH measurement apparatus 10 of fiber probe type, as illustrated in Figure 7, is used, an examiner can directly move the probe to measure pH at an arbitrary region. Therefore, when an abnormal region detection means 80 that can detect pH distribution of the substance D to be measured is provided in a manner similar to the pH measurement apparatus 1, the pH measurement apparatus 10 can function as an abnormal region detection apparatus 11.

In this embodiment, an MEMS (micro-electro mechanical system) scanner, which is attached to the leading end of a fiber probe during use, or the like may be used as the abnormal region detection means 80. Further, it is possible to perform image detection in a desirable manner by providing an operation unit 91 for the stage 90 on which the substance to be measured is placed.

The abnormal region detection apparatus 2 (2') of the present embodiment includes the pH measurement apparatus of the above embodiment. Therefore, the abnormal region detection apparatus 2 (2') can achieve an advantageous effect similar to the pH measurement apparatus 1 (1').

"Living Mater Analysis Apparatus"

With reference to Figures 1, 5, 6 and 7, living matter analysis apparatus 3 (6, 9 and 12) of the present embodiment will be described. The living matter analysis apparatus 3 (6, 9 and 12) is structured by providing an analysis means 70 for the abnormal region detection apparatus 2 (5, 8 and 11). The analysis means 70 identifies, based on measured pH, a pathological condition of the substance D to be measured.

It is known that when a living tissue becomes abnormal, the pH in the tissue changes. For example, when a tissue is cancerized (malignant tumor), or when a disease, such as a liver disease, is present, the value of pH becomes low. Therefore, it is possible to identify a pathological condition, such as whether a malignant tumor condition is present, and the degree of malignant condition at a predetermined position or region in the substance D to be measured by using the pH measurement apparatus 1 (4, 7 and 10) or the abnormal region detection apparatus 2 (5, 8 and 11).

In the structure of the present embodiment, it is possible to obtain the in-plane pH distribution of the substance D to be measured. Therefore, it is possible to identify an abnormal range of the pathological condition with respect to the in-plane direction of the substance D to be measured. Further, it is possible to identify the degree of the abnormal condition in the range.

In treatment of malignant tumor (cancer), there are surgical therapy, radiation therapy, and the like. In the surgical therapy, an affected part is extracted by excision. Meanwhile, in the radiation therapy, the affected part is not removed, and cancer cells are killed by irradiating the affected part with radiation. Since the living matter analysis apparatus of the present embodiment can identify a cancerized range, it is possible to accurately remove only a region that needs to be removed in the surgery therapy. In surgery for extracting a cancerous tumor, it is not desirable that cancer cells remain in a patients body after surgery, because the risk of recurrence increases. However, if tissue is excessively removed because of the fear of recurrence, a burden on the patient increase, and that is not desirable, either. Therefore, only a region that is judged to be necessary to be removed is extracted. After extracting the region, a very small amount of tissue in a region that was in contact with the extracted tumour is removed, and examination is performed on the tissue by using the pH measurement apparatus of the present invention to find whether a cancer cell is present in the tissue. Alternatively, the extracted tumor is used as a substance to be measured (sample), and pH measurement is performed on the cut surface of the sample by using the pH measurement apparatus to find whether a cancer cell is present on the cut surface. In either case, if a cancer cell is detected, it is judged that further excision is necessary. If no cancer cell is detected, it is judged that a sufficient region has been removed.

Further, in radiation therapy, it is possible to accurately recognize a region that is necessary to be irradiated with radiation. Therefore, it is possible to minimize a part of the patient's body damaged by radiation.

As described above, the living matter analysis apparatus of the present embodiment includes the pH measurement apparatus that can highly accurately measure pH. Therefore, it is possible to obtain highly accurate pH information about an affected part, such as a cancerous region, together with the position information about the affected part. Therefore, according to the living matter analysis apparatus of the present embodiment, it is possible to select the type of radiation, the intensity or dose of radiation in an appropriate manner based on the condition of the affected part in radiation therapy. Hence, an excellent treatment result is achievable. Further, in endoscopic submucosal dissection (ESD), it is possible to identify the range and the condition of an affected part. Therefore, it is possible to remove an appropriate region, and to minimize a damage to the patient.

Further, it is necessary to quickly judge whether a cancer should be removed during surgery. Since the pH measurement apparatus of the present embodiments can measure pH immediately and quickly, as described above, the throughput of the pH measurement apparatus is high, and the pH measurement apparatus is desirable as an analysis apparatus for determining a treatment policy of cancer.

Meanwhile, each cell or tissue has different cell environment, because the same normal cell or cancer cell may be present in different conditions, such as an activity state, a stress state and a weak state. Therefore, the lifetime of autofluorescence from a cell or tissue varies depending on the environment of each cell or tissue.

In actual measurement on an affected part, it is necessary to generate a calibration curve based on a sufficient amount of clinical data. Further, it is necessary to obtain more accurate data about a region to be measured by repeating measurement on different measurement regions. When measurement is performed in such a manner, it is possible to perform more accurate measurement of pH on cells or tissues that vary greatly and to make more accurate clinical judgments.

As an example, pH measurement was performed on a border region between a cancer cell and a normal cell of a cancer-bearing mouse. Here, autofluorescent material to be excited is NADH and NADPH, and SHG light of a titanium sapphire laser with a pulse width of 1 picosecond was used as excitation light. Further, the wavelength of the excitation light was adjusted to 370 nm, which is the excitation wavelength of the NADH and NADPH (only reduction type is excited). Further, a fluorescence filter of 417 through 477 nm was used to measure fluorescence lifetime. At this time, the average power of the excitation light was 0.9 mW, and pulse energy was 12 pJ. A predetermined position in the border region between the cancer tissue and the normal tissue was illuminated with laser light, and the position of the xyz stage was adjusted so that the laser light was focused on the border region between the cancer tissue and the normal tissue.

The calibration curve was prepared based on sufficient clinical data. Three regions of cancer tissue and three regions of normal tissue were separately measured, and average values of fluorescence lifetime were obtained based on three kinds of data for the cancer tissue and three kinds of data for the normal tissue, respectively. The obtained average values were compared with the calibration curve, and the pH of the cancer cell was 7.1, and the pH of the normal cell was 7.2. However, when ten regions of cancer tissue and ten regions of normal tissue were separately measured, and average values of fluorescence lifetime were obtained based on ten kinds of data for the cancer tissue and ten kinds of data for the normal tissue, respectively, the pH of the cancer cell was 6.8, and the pH of the normal cell was 7.3. The difference in the pH values between 6.8 and 7.3, which is 0.5, is greater than pH resolution by fluorescence lifetime, which is a value in the range of from 0.3 to 0.4. As described above, it is possible to perform more accurate measurement and to make more accurate clinical judgments by increasing the number of times of measurement.

"Design Modification"

So far, embodiments of the pH measurement apparatus, the abnormal region detection apparatus including the pH measurement apparatus and the living matter analysis apparatus according to the present invention have been described. However, the present invention is not limited to the embodiments, and various modifications are possible without deviating from the gist of the present invention.

Identification of the pathological condition of substance D to be measured was described with respect to the structure using the analysis means 70. However, when a relationship between pH and the degree of abnormality of the pathological condition is clear, it is possible to identify the pathological condition of living matter by using only the pH measurement apparatus and the abnormal region detection apparatus in the aforementioned embodiments. Therefore, the result of treatment described with respect to the living matter analysis apparatus is achievable also by the pH measurement apparatus and the abnormal region detection apparatus in the above embodiments.

"Examples"

Next, examples of the present invention will be described.

(Example 1)

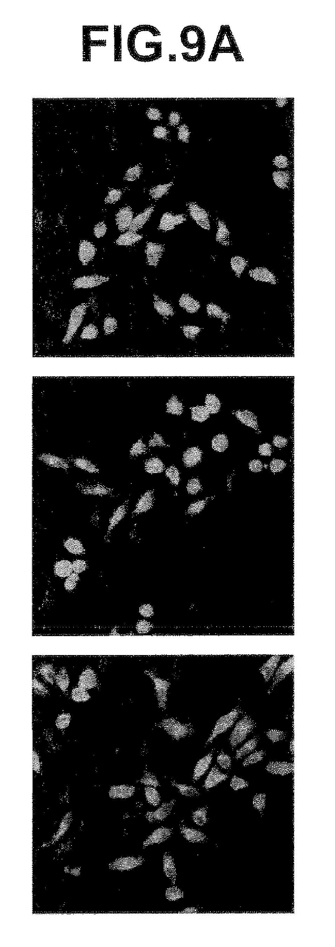

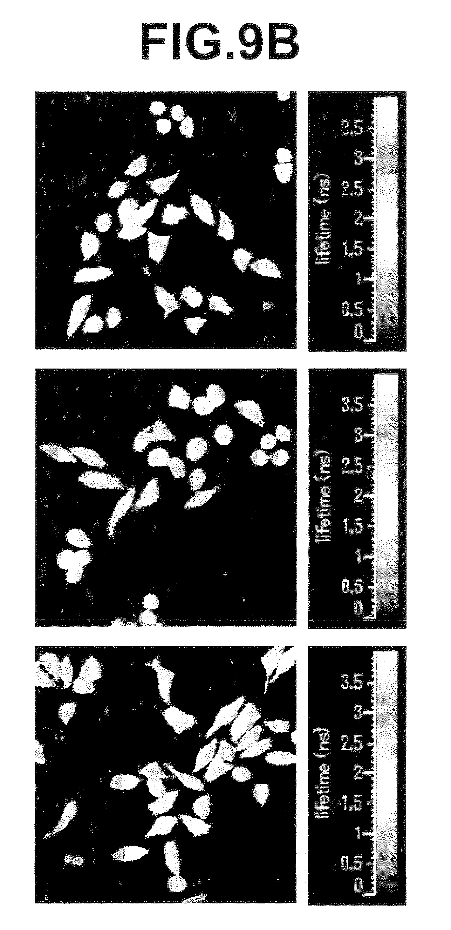

The pH of a HeLa cell that had been cultured, and the pH of which had not been adjusted, was measured by using the microscope-type pH measurement apparatus and the abnormal region detection apparatus, illustrated in Figure 1. Figure 9A is a diagram illustrating a fluorescence intensity image of the measured HeLa cell, and Figure 9B is a diagram illustrating a fluorescence lifetime image of the HeLa cell. Measurement was performed three times on a sample in the same conditions at different measurement regions. The average fluorescence lifetime of three entire images was 2.24 nsec. When pH was calculated based on the obtained fluorescence lifetime by using the equation (1), the pH of the HeLa cell was estimated to be 6.6.

industrial Applicability"

The pH measurement method, the abnormal region detection method, and the living matter analysis method of the present invention and the apparatuses for carrying out the methods are desirably used in diagnosis as to whether a cell is a normal cell or an abnormal cell (cancer cell or the like) and in separation of the normal cell and the abnormal cell from each other.

**Claims**

1. A pH measurement method comprising the steps of:

   generating pulsed excitation light (L1) including a wavelength that can excite a predetermined fluorescent material contained in living matter, the fluorescent material acting as coenzyme in oxidation/reduction reaction in vivo, and the intensity of the pulsed excitation light (L1) not damaging a tissue nor a cell in the living matter and substantially not changing the pH of the living matter;
   illuminating a predetermined position in the living matter with the pulsed excitation light (L1);
   receiving light including fluorescence (Lf) emitted from the fluorescent material excited by illumination with the pulsed excitation light (L1);
   calculating the lifetime (Tf) of the fluorescence (Lf) by time-resolving the intensity of the received fluorescence (Lf); and
   measuring the pH of the living matter based on the lifetime (Tf).

2. A pH measurement method, as defined in Claim 1, wherein the lifetime (Tf) of fluorescence emitted from the fluorescent material changes at least by 0.03 nanosecond in the range of from pH 6.5 to 7.5.

3. A pH measurement method, as defined in Claim 1 or 2, wherein the fluorescent material is at least one kind of fluorescent material selected from the group consisting of NADH, NADPH and FAD.

4. A pH measurement method, as defined in any one of Claims 1 to 3, wherein the living matter is cytoplasm, a mitochondrion and a nucleus.

5. A pH measurement method, as defined in any one of Claims 1 to 4, wherein the lifetime (Tf) of the received fluorescence (Lf) is calculated for each wavelength by wavelength-resolving the fluorescence (Lf) to obtain fluorescence spectrum (Ls) of the fluorescence and by time-resolving, based on the fluorescence spectrum (Ls) the intensity of the fluorescence (Lf) for the respective wavelengths.

6. A pH measurement method, as defined in any one of Claims 1 to 5, wherein the fluorescence (Lf) is excited by multiphoton excitation.

7. A pH measurement method, as defined in any one of Claims 1 to 6, wherein the predetermined position is a plurality of positions.

8. A detection method comprising the steps of:

measuring the pH of the predetermined position by using the pH measurement method, as defined in any one of Claims 1 to 7, when the predetermined position is evenly distributed in a predetermined region of the living matter; and
detecting an abnormal region in the predetermined region of the living matter based on the obtained pH of the predetermined position.

9. A detection method, as defined in Claims 8, wherein the abnormal region in the living matter is detected by generating and displaying an image of the abnormal region.

10. A living matter analysis method, wherein the pathological condition of the living matter is identified based on the pH measured by using the pH measurement method, as defined in any one of Claims 1 to 7.

11. A living matter analysis method, wherein the abnormal region is detected by using the detection method, as defined in Claim 8 or 9, and the pathological condition of the predetermined region is identified.

12. A living matter analysis method, as defined in Claim 10 or 11, wherein the pathological condition is presence of malignant tumor condition.

13. A pH measurement apparatus (1, 4, 7, 10) comprising:

an excitation light generation means (10) that generates pulsed excitation light (L1) including a wavelength that can excite a predetermined fluorescent material contained in living matter, the fluorescent material acting as coenzyme in oxidation/reduction reaction in vivo, and the intensity of the pulsed excitation light (11) not damaging a tissue nor a cell in the living matter and substantially not changing the pH of the living matter;
an excitation light illumination means (20) that illuminates a predetermined position in the living matter with the pulsed excitation light (L1);
a light receiving means (30) that receives light including fluorescence (Lf) emitted from the fluorescent material excited by illumination with the pulsed excitation light (L1);
a time-resolving means (40) that time-resolves the fluorescence (Lf) synchronously with illumination with the pulsed excitation light (L1);
a detection means (50) that detects the time-resolved fluorescence (Lf); and
a measurement means (60) that calculates the lifetime (Tf) of the fluorescence (Lf) based on the time-resolved fluorescence (Lf) detected by the detection means (50) and measures the pH of the living matter based on the lifetime (Tf).

14. A pH measurement apparatus (1, 4, 7, 10), as defined in Claim 13, further comprising:

a spectral means that separates the fluorescence (Lf) received by the light receiving means (30) to obtain wavelength-resolved fluorescence (Lf), and outputs the wavelength-resolved fluorescence (Lf) to the time-resolving means (40).

15. A pH measurement apparatus (1, 4, 7, 10), as defined in Claim 13 or 14, wherein the fluorescent material is at least one kind of fluorescent material selected from the group consisting of NADH, NADPH and FAD.

16. A pH measurement apparatus (1, 4, 7, 10), as defined in any one of Claims 13 to 15, wherein the living matter is cytoplasm, a mitochondrion and a nucleus.

17. A pH measurement apparatus (1, 4, 7, 10), as defined in any one of Claims 13 to 16, wherein the fluorescence (Lf) is excited by multi-photon excitation.

18. A pH measurement apparatus (1, 4, 7, 10), as defined in Claim 17, wherein the excitation light generationmeans (10) includes a laser (11) that emits pulses with a pulse width in the range of from a femtosecond to hundreds of picoseconds.

19. A pH measurement apparatus (1, 4, 7, 10), as defined in any one of Claims 13 to 18, further comprising:

a stage (90, 90') that keeps the living matter in contact with a surface of the stage (90, 90'), and which is movable in three-dimensional directions so that an arbitrary position in the living matter is illuminated with the pulsed excitation light (Ll); and
a position adjustment means (91, 91') that moves the stage (90, 90') to an arbitrary position in three-dimensional directions,
wherein the excitation light illumination means (20) includes an optical system (21, 31) that receives the pulsed excitation light (L1) and illuminates the living matter with the pulsed excitation light (L1), and
wherein the light receiving means (30) includes an optical system (21, 31) that receives the light including the fluorescence (Lf) emitted from the fluorescent material excited by illumination with the pulsed excitation light (L1), and that guides the light to the time-resolving means (40).

20. A pH measurement apparatus (1, 4, 7, 10), as defined in any one of Claims 13 to 19, further comprising:

a stage (90, 90') that keeps the living matter in contact with a surface of the stage (90, 90'), and which is movable so that an arbitrary position in the living matter, at least in the direction of an optical axis of the pulsed excitation light (L1), is illuminated with the pulsed excitation light (L1);
a first position adjustment means (91, 91') that moves the stage (90, 90') to an arbitrary position at least in the direction of the optical axis; and
a second position adjustment means (91, 91') that moves the excitation light illumination means (20) so that an arbitrary position at least in an in-plane direction, which is perpendicular to the optical axis of the pulsed excitation light (L1), in the living matter is illuminated with the pulsed excitation light (L1),
wherein the excitation light illumination means (20) includes an optical system (21, 31) that receives the pulsed excitation light (L1) and illuminates the living matter with the pulsed excitation light (L1), and
wherein the light receiving means (30) includes an optical system (21, 31) that receives the light including the fluorescence (Lf) emitted from the fluorescent material excited by illumination with the pulsed excitation light (L1), and that guides the light to the time-resolving means (40) .

21. A pH measurement apparatus (1, 4, 7, 10), as defined in any one of Claims 13 to 19, further comprising:

a position adjustment means (91, 91') that moves the excitation light illumination means (20) so that an arbitrary position in three-dimensional directions in the living matter is illuminated with the pulsed excitation light (L1),
wherein the excitation light illumination means (20) includes an optical system (21, 31) that receives the pulsed excitation light (L1) and illuminates the living matter with the pulsed excitation light (L1), and
wherein the light receiving means (30) includes an optical system (21, 31) that receives the light including the fluorescence (Lf) emitted from the fluorescent material excited by illumination with the pulsed excitation light (L1), and that guides the light to the time-resolving means (40).

22. A pH measurement apparatus (1 4, 7, 10), as defined in any one of Claims 13 to 21, wherein the excitation light illumination means (20) includes at least one optical fiber (200) for illumination that illuminates the living matter with the pulsed excitation light (L1), and
wherein the light receiving means (30) includes at least one optical fiber (300) for receiving light that receives the light including the fluorescence (Lf) emitted from the fluorescent material excited by illumination with the excitation light (L1) and that guides the fluorescence (Lf) to the time-resolving means (40) .

23. A pH measurement apparatus (1, 4, 7, 10), as defined in Claim 22, wherein the at least one optical fiber (200) for illumination and the at least one optical fiber (300) for receiving light form a bundle fiber (21', 31').

**24.** A pH measurement apparatus (1, 4, 7, 10), as defined in Claim 23, wherein the bundle fiber (21', 31') is formed by bundling an optical fiber (200) for illumination and a plurality of optical fibers (300) for receiving light together in such a manner that the outer surface of the optical fiber (200) for illumination arranged substantially at the center of the bundle fiber (21', 31') is surrounded by the plurality of optical fibers (300) for receiving light.

**25.** A pH measurement apparatus (1, 4, 7, 10), as defined in Claim 23 or 24, wherein the bundle fiber (21', 31') is a fiber probe (F) that is provided in a substantially-cylindrical long sheath to be inserted into body cavity.

**26.** A pH measurement apparatus (1, 4, 7, 10), as defined in Claim 23 or 24, wherein the bundle fiber (21', 31') is provided in a forceps channel (101) of an endoscope (100) that includes an illumination unit (102) for illuminating a predetermined position in body cavity with illumination light, an imaging unit (103) that images reflection light reflected from the predetermined position, and the forceps channel (101).

**27.** A pH measurement apparatus (1, 4, 7, 10), as defined in Claim 25, wherein the fiber probe (F) is provided in a forceps channel (101) of an endoscope (100) that includes an illumination unit (102) for illuminating a predetermined position in body cavity with illumination light, an imaging unit (103) that images reflection light reflected from the predetermined position, and the forceps channel (101) in such a manner that the fiber probe (F) projects from an opening of the forceps channel (101) on the predetermined position side.

**28.** A pH measurement apparatus (1, 4, 7, 10), as defined in any one of Claims 13 to 27, wherein the predetermined position is a plurality of positions.

**29.** A detection apparatus (2, 5, 8, 11) comprising:

a pH measurement apparatus (1, 4, 7, 10), as defined in Claim 28, when the plurality of positions are evenly distributed in a predetermined region of the living matter; and
an abnormal region detection means (80) that detects an abnormal region in the predetermined region of the living matter based on the values of pH of the plurality of positions measured by the pH measurement apparatus (1, 4, 7, 10).

**30.** A detection apparatus (2, 5, 8, 11), as defined in Claim 29, further comprising:

a display device that generates and displays an image of the abnormal region detected by the abnormal region detection means (80) .

**31.** A living matter analysis apparatus (3, 6, 9, 12) comprising:

a pH measurement apparatus (1, 4, 7, 10) as defined in any one of Claims 13 to 28; and
an analysis means (70) that identifies the pathological condition of the living matter based on the pH measured by the pH measurement apparatus (1, 4, 7, 10).

**32.** A living matter analysis apparatus (3, 6, 9, 12), as defined in Claim 31, wherein the pathological condition is presence of malignant tumor condition.

**33.** A living matter analysis apparatus (3, 6, 9, 12 ) , as defined in Claim 31 or 32, further comprising:

an abnormal region detection means (80) that detects an abnormal region in a predetermined region of the living matter based on the value of pH measured by the pH measurement apparatus (1, 4, 7, 10); and
an analysis means (70) that identifies the pathological condition of the abnormal region detected by the abnormal region detection means (80).

# FIG.1

# FIG.2

# FIG.3A

# FIG.3B

# FIG.4

EXCITATION LIGHT PULSE

FLUORESCENCE

$$I = I_0 e^{-t/Tf}$$

FLUORESCENCE INTENSITY(I)

$I_0$

$1/e$

$t_1$  $t_2$  $t_3$

TIME (ns)

# FIG.5

# FIG.6

EP 2 365 337 A1

# FIG.7

24

# FIG.8A

100

102  103

104

102

21'(31')

101

# FIG.8B

100'

102  103

104

102

21'(31')

F

101

# FIG.9A

# FIG.9B

## EUROPEAN SEARCH REPORT

Application Number

EP 11 15 7638

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2007/083138 A1 (PERKINELMER SINGAPORE PTE LTD [SG]; COURTNEY PATRICK [GB]; ORANGE PAUL) 26 July 2007 (2007-07-26) | 1-3,6, 13-21,28 | INV. G01N33/84 |
| Y | * page 5, paragraph 4 * <br> * page 6, paragraph 2 * <br> * page 13 - page 14 * <br> * figures 7,8,10 * | 4,5, 8-12,16, 18, 22-27, 29-33 | |
| Y | US 2004/073119 A1 (MYCEK MARY-ANN [US] ET AL) 15 April 2004 (2004-04-15) <br><br> * paragraphs [0004], [0006], [0014], [0021], [0035] * | 8-12, 22-27, 29-33 | |
| Y | YU QIANRU ET AL: "Integrated biophotonics approach for noninvasive and multiscale studies of biomolecular and cellular biophysics", <br> JOURNAL OF BIOMEDICAL OPTICS, <br> vol. 13, no. 4, 041315, <br> July 2008 (2008-07), pages 1-14, <br> XP002636886, <br> ISSN: 1083-3668 <br> * page 2 - page 13 * | 4,16,18 | TECHNICAL FIELDS SEARCHED (IPC) <br><br> G01N |
| Y | DE BEULE P ET AL: "A hyperspectral fluorescence lifetime probe for skin cancer diagnosis", <br> REVIEW OF SCIENTIFIC INSTRUMENTS, <br> vol. 78, no. 12, 123101, <br> 4 December 2007 (2007-12-04), pages 1-7, <br> XP012105546, <br> AIP, MELVILLE, NY, US <br> ISSN: 0034-6748, DOI: 10.1063/1.2818785 <br> * figure 5 * | 5 | |

-/--

The present search report has been drawn up for all claims

| Place of search | Date of completion of the search | Examiner |
|---|---|---|
| The Hague | 18 May 2011 | Chrétien, Eva Maria |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

Europäisches Patentamt
European Patent Office
Office européen des brevets

# EUROPEAN SEARCH REPORT

Application Number

EP 11 15 7638

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 92/13265 A1 (UNIV MARYLAND [US]; NOWACZYK KAZIMIERZ [US]; SZMACINSKI HENRYK [US]; J) 6 August 1992 (1992-08-06)<br>* examples 1-4 *<br>* claims 1,7,27-35 * | 1-33 | |
| A | HANSON KERRY M ET AL: "Two-photon fluorescence lifetime imaging of the skin stratum corneum pH gradient",<br>BIOPHYSICAL JOURNAL,<br>vol. 83, no. 3, September 2002 (2002-09), pages 1682-1690, XP002636887,<br>ISSN: 0006-3495<br>* the whole document * | 1-33 | |
| A | US 5 648 269 A (LAKOWICZ JOSEPH R [US] ET AL) 15 July 1997 (1997-07-15)<br>* the whole document * | 1-33 | |
| A | YU Q ET AL: "Two-photon autofluorescence dynamics imaging reveals sensitivity of intracellular NADH concentration and conformation to cell physiology at the single-cell level",<br>JOURNAL OF PHOTOCHEMISTRY AND PHOTOBIOLOGY B: BIOLOGY,<br>vol. 95, no. 1, 2 April 2009 (2009-04-02), pages 46-57, XP025981402,<br>ELSEVIER SCIENCE S.A., BASEL, CH<br>ISSN: 1011-1344, DOI: 10.1016/J.JPHOTOBIOL.2008.12.010<br>[retrieved on 2009-03-14]<br>* the whole document * | 1-33 | TECHNICAL FIELDS SEARCHED (IPC) |

-/--

The present search report has been drawn up for all claims

| Place of search | Date of completion of the search | Examiner |
|---|---|---|
| The Hague | 18 May 2011 | Chrétien, Eva Maria |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

**Application Number**

EP 11 15 7638

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | MARTIN OHEIM ET AL: "Multiparameter Evanescent-Wave Imaging in Biological Fluorescence Microscopy", IEEE JOURNAL OF QUANTUM ELECTRONICS, vol. 38, no. 2, 1 February 2002 (2002-02-01), XP011052771, IEEE SERVICE CENTER, PISCATAWAY, NJ, USA ISSN: 0018-9197 * the whole document * | 1-33 | |
| A | LAKOWICZ J R ET AL: "Fluorescence lifetime imaging of free and protein-bound NADH.", PROCEEDINGS OF THE NATIONAL ACADEMY OF SCIENCES OF THE UNITED STATES OF AMERICA, vol. 89, no. 4, 15 February 1992 (1992-02-15), pages 1271-1275, XP002636888, ISSN: 0027-8424 * the whole document * | 1-33 | |
| A | VAN MUNSTER ERIK B ET AL: "Fluorescence lifetime imaging microscopy (FLIM)", ADVANCES IN BIOCHEMICAL ENGINEERING, BIOTECHNOLOGY, vol. 95, 1 January 2005 (2005-01-01), pages 143-175, XP008105148, SPRINGER, BERLIN, DE ISSN: 0724-6145, DOI: 10.1007/B102213 [retrieved on 2005-06-02] * the whole document * | 1-33 | **TECHNICAL FIELDS SEARCHED (IPC)** |

The present search report has been drawn up for all claims

| Place of search | Date of completion of the search | Examiner |
|---|---|---|
| The Hague | 18 May 2011 | Chrétien, Eva Maria |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.**

EP 11 15 7638

This annex lists the patent family members relating to the patent documents cited in the above-mentioned European search report.
The members are as contained in the European Patent Office EDP file on
The European Patent Office is in no way liable for these particulars which are merely given for the purpose of information.

18-05-2011

| Patent document cited in search report | | Publication date | Patent family member(s) | | Publication date |
|---|---|---|---|---|---|
| WO 2007083138 | A1 | 26-07-2007 | AU 2007206707 A1 | | 26-07-2007 |
| | | | CA 2635238 A1 | | 26-07-2007 |
| | | | EP 1974203 A1 | | 01-10-2008 |
| | | | JP 2009524040 T | | 25-06-2009 |
| | | | KR 20080090515 A | | 08-10-2008 |
| | | | US 2009173892 A1 | | 09-07-2009 |
| US 2004073119 | A1 | 15-04-2004 | NONE | | |
| WO 9213265 | A1 | 06-08-1992 | EP 0568596 A1 | | 10-11-1993 |
| | | | US 5485530 A | | 16-01-1996 |
| US 5648269 | A | 15-07-1997 | NONE | | |

EPO FORM P0459

For more details about this annex : see Official Journal of the European Patent Office, No. 12/82

**EP 2 365 337 A1**

**Patent documents cited in the description**

- JP 2000139462 A
- JP 2001523334 PCT
- JP 2003512085 PCT